# EUROPEAN PATENT APPLICATION

(11) **EP 1 743 946 A1**
(43) Date of publication of application: **17.01.2007**
(21) Application number: 04805141.1
(22) Date of filing: 03.12.2004
(51) Int. Cl.: C12Q 1/68

(54) **METHOD AND KIT FOR THE GENOTYPIFICATION OF HLA-DRB BASED ON REAL TIME PCR**

(30) Priority: 17.12.2003 ES 200400010
(71) Applicant: UNIVERSITAT AUTONOMA DE BARCELONA, 08193 Bellaterra (ES)
(72) Inventor: JUAN OTERO, Manuel, LIRAD - SSR-CTBT, 08916 Badalona (Barcelona) (ES); CASAMITJANA PONCES, Natàlia, LIRAD - SSR-CTBT, 08916 Badalona (Barcelona) (ES); PUJOL BORRELL, Ricardo, LIRAD - SSR-CTBT, 08916 Badalona (Barcelona) (ES); COLOBRAN ORIOL, Roger, LIRAD - SSR-CTBT, 08916 Badalona (Barcelona) (ES); PALOU RIVERA, Eduardo, LIRAD - SSR-CTBT, 08916 Badalona (Barcelona) (ES); FANER CANET, Maria Rosa, LIRAD - SSR-CTBT, 08916 Badalona (Barcelona) (ES); RIBERA CRUSAFONT, Ana, LIRAD - SSR-CTBT, 08916Bad alona (Barcelona) (ES)
(74) Representative: Barlocci, Anna
(86) International application number: PCT/ES2004/070104
(87) International publication number: WO 2005/059174

(57) **Abstract**

Method for determining the genotype of loci DRB of human leukocyte antigen from a nucleic acid sample, and kit for performing this method. The kit comprises allele-specific primers for amplifying nucleic acids derived from HLA-DRB loci by real-time PCR; allele-specific fluorescently labelled nucleotide probes for detecting fluorescence signals during the amplification; and experimentally defined fluorescence patterns for comparing the detected signals. In particular the genes of DRB loci are selected from DRB1, DRB3, DRB4 and DRB5. It overcomes some of the limitations of conventional DNA-based typing methods, in terms of time, possibility of automation, and increase in resolution, while reducing the number of PCR reactions. It is useful e.g. for determining transplantation compatibility or susceptibility for a specific disease.

## Description

This invention relates to the field of medicine generally and specifically to medical research, molecular biology, forensics and diagnostics. In particular, the invention provides a method for determining the genotype (i.e. the specific set of alleles) of an individual. This facilitates determining the transplantation compatibility and the susceptibility for a specific disease of the individual.

### BACKGROUND ART

In transplantation between immunogenetically different individuals, transplantation immunity is established and a rejection reaction is induced against the graft. There are antigens inducing particularly intense transplantation immunity as targets in this reaction, which are called major histocompatibility antigens. These antigens are controlled by a group of genes called major histocompatibility complex (MHC), known in human as human leukocyte antigens (HLA) system. The genes that encode the HLA molecules are among the most variable genes in humans, each variant coding for molecules which bind to different set of peptides. These genes are the same in all the cells of a particular individual, but differ from person to person.

HLA comprises several genes clustered on a 4 Mb-segment of the short arm of chromosome 6 (cf. D.A. Rhodes et al., "Genetics and molecular genetics of the MHC", Rev. Immunogenet. 1999, vol. 1, pp. 21-31). The HLA region comprises six major loci that encode structurally homologous proteins which are classified into HLA class I (HLA-A, B, Cw) and class II (HLA-DR, DQ, DP), that present antigens to two different subsets of cells. Class II molecules are heterodimeric glycoproteins consisting of a non-polymorphic alpha chain and a polymorphic beta chain. Class II MHC molecules are expressed on the cell surface of macrophages, B-cells, activated T-cells, and other cell types involved in presenting antigens to T cells that express the CD4 cell surface glycoprotein (cf. Bjorkman et al., Ann. Rev. Biochem. 1990, vol. 59, pp. 253-88). DP, DQ and DR genes are located in separate regions of the MHC. In the DR region, a single DRA locus encodes the non-polymorphic DRalpha chain, but nine different DRB loci, termed DRB1 to DRB9, encode the polymorphic DRbeta chain. The number of DRB alleles identified is continually increasing. Due to this fact, accurate determination of alleles is essential for typing potential transplantation donors, where very precise HLA matching of the donor and the transplant recipient appears to be critical in minimizing risk of rejection.

Serological tests using antiserum reactions and cytological tests have been generally used for HLA typing. However, long time and extensive labor are required for examination, operation is complicated, and accuracy of obtainable results is rather low. HLA typing by techniques based on polymerase chain reaction (PCR) have become a widely used alternative to serological methods in clinical practice. The most common PCR-based HLA typing methods that overcome serological tests limitations, are PCR with sequence-specific primers (PCR-SSP) (cf. O. Olerup et al., "HLA-DR typing by PCR amplification with sequence specific primers (PCR-SSP) in 2 hours: an alternative to serological DR typing in clinical practice including donor-recipient matching in cadaveric transplantation", Tissue Antigens 1992, vol. 39, pp. 225-35; M. Bunce et al., "Phototyping : comprehensive DNA typing for HLA-A, B, C, DRB1, DRB3, DRB4, DRB5 & DQB1 by PCR with 144 primer mixes utilizing sequence-specific primers (PCR-SSP)", Tissue Antigens 1995, vol. 46, pp. 355-67) and PCR-SSO (sequence-specific oligonucleotide hybridization) (cf. EP 514.534; EP 417.160 and R.K. Saiki et al., "Analysis of enzymatically amplified beta-globin and HLA-DQ alpha DNA with allele-specific oligonucleotide probes", Nature 1986, vol. 324, pp. 163-6).

In PCR-SSP method, gene sequence is confirmed by amplifying the hypervariable region of target HLA antigen to determine the HLA antigen type.
In PCR-SSO method, a nylon membrane (on which DNA amplified by primers specific to HLA gene is immobilized) is prepared and respective HLA type specific oligonucleotide probes are hybridized for typing. While both methods have been effective in the routine laboratory practice, they require time-consuming post-PCR processing, and post-PCR contamination is a real risk. Moreover, PCR-SSP requires the use of a large number of PCR reactions and it is difficult to automate, thus limiting its throughput. On the other hand, even if PCR-SSO is capable of a higher throughput, its processing times are longer and it can not discriminate among motifs occurring on cis-trans strands of the DNA.

HLA typing laboratories have to analyze a large number of samples and need to increase typing resolution in order to have more feasible clinical results for transplantation, particularly bone marrow transplantation. Thus, it would be highly desirable to provide new typing methods, particularly if they are improved in the sense of they are faster, are potentially automated, give high resolution and reduce contamination risks.

### SUMMARY OF THE INVENTION

Some previous studies have reported that real-time PCR based HLA typing, also called fluorotyping, does not require post-PCR processing and could be fully automated (cf. EP 1.229.128 A1; S.J. Faas et al., "Sequence-specific priming and exonuclease-released fluorescence detection of HLA-DQB1 alleles", Tissue Antigens 1996, vol. 48, pp. 97-112; K. Slateva et al., "HLA-DRB fluorotyping by dark quenching and automated analysis", Tissue Antigens 2001, vol. 58, pp. 250-4). In the previous art real-time PCR to HLA typing only was used as an equivalent to PCR-SSP with a fluorogenic locus-specific probe. Further expanding the usefulness of real-time PCR in the present invention this technology is used to discriminate among the alleles of a given locus.

Thus, an aspect of the present invention relates to a method for determining the HLA-DRB genotype from a nucleic acid sample, comprising the steps of: (i) amplifying any nucleic acid that comprises exon 2 of at least one of the genes of the DRB loci by real-time PCR; (ii) detecting fluorescence signals by fluorescently labelled nucleotide probes during the amplification carried out in step (i), under conditions where said probes specifically bind to the amplified nucleic acid sequences; and (iii) comparing more than one signal detected in step (ii) with an experimentally defined fluorescence pattern; said pattern having been established by an initial definition based on theoretical comparison of the sequences of nucleotide probes of step (ii) with the sequences of the different alleles of the selected genes of step (i), followed by a definitive definition based on an experimental assessment of which of the theoretical signals are actually positive (black squares in FIG. 2) and which are actually negative (white squares in FIG. 2). Steps (i) and (ii) of this method are carried out in the same tube and without any further manipulation of the sample.

In general, the nucleic acid in the sample will be DNA, most usually genomic DNA. However, the method of the present invention can also be practiced with other nucleic acids, such as messenger RNA or cloned DNA, and the nucleic acid may be either single-stranded or double-stranded.

Another aspect of the present invention relates to a kit for determining the HLA-DRB genotype from a nucleic acid sample by performing the method described above. This kit comprises: (i) primers for amplifying nucleic acids of at least one of the genes of the HLA-DRB loci by real-time PCR; (ii) fluorescently labelled nucleotide probes for detecting fluorescence signals during the amplification; and (iii) the experimentally defined fluorescence patterns for comparing the detected signals as is described above. In a particular embodiment of this invention, the genes of the DRB loci in step (ii) are selected from the group consisting of DRB1, DRB3, DRB4 and DRB5.

Real-time PCR monitors the fluorescence emitted during the reaction as an indicator of amplimer production during each PCR cycle (i.e., in real time), as opposed to the endpoint detection by conventional quantitative PCR methods. The real-time PCR does not detect the size of the amplimer and thus does not allow the differentiation between DNA and cDNA amplification. The real-time PCR system is based on the detection and quantification of a fluorescent reporter.

In a particular embodiment of the invention, the kit provides primers which are single strand nucleic acids which have a length from 10 to 30 nucleotides, particularly from 14 to 24 nucleotides, and which comprise sequences complementary to sequences of a region located along intron 1-exon 2-intron 2 of at least one of the genes selected from the group consisting of DRB1, DRB3, DRB4 and DRB5. Particularly, primer sequences are selected from the group consisting of SEQ ID NO: 1-19. The amplifying step (i) of the method is carried out with two or more primers. Real-time PCR is performed using universal cycling thermal parameters and PCR reaction conditions known for a skilled in the art.

The kit of the invention also provides probes which are single strand nucleic acids which have a length from 10 to 45 nucleotides, particularly from 13 to 34 nucleotides, and which comprise sequences complementary to sequences of a region located along exon 2 of at least one of the genes selected from the group consisting of DRB1, DRB3, DRB4 and DRB5. Particularly, probe sequences are selected from the group consisting of SEQ ID NO: 22-48 or from the complementary sequences of this group. In a particular embodiment, probes are labelled with fluorochromes of type reporter label-quencher label, and probes are degraded due to the 5'->3' exonuclease activity of DNA polymerase. In particular, TaqMan probes from Applied Biosystems, Foster City, CA, USA, are used to this end. Specific label for this purpose are FAM (6-carboxy-fluorescein), VIC and TET (6-carboxy-4,7,2',7'-tetrachloro-fluorescein), which are fluorescent reporter labels, and TAMRA (6-carboxy-N,N,N',N'-tetramethyl-rhodamine) and MGB (DNA minor groove binder) which are fluorescent and non-fluorescent quencher labels, respectively. Taqman probes use the fluorogenic 5' exonuclease activity of Taq polymerase to measure the amount of target sequences in samples. This probes are primers longer than the primers (20-30 bases long with a Tm value of 10 °C higher) that contain a fluorescent label usually on 5', and a quenching label (usually TAMRA) typically on 3'. When irradiated, the excited fluorescent label transfers energy to the nearby quenching label molecule rather than fluorescing (this is called FRET, acronym of "Förster or fluorescence resonance energy transfer"). Thus, the proximity of the reporter and quencher prevents emission of any fluorescence while the probe is intact. TaqMan probes are designed to anneal to an internal region of a PCR product. When the polymerase replicates, its 5' exonuclease activity cleaves the probe. This ends the activity of quencher (no FRET) and the reporter label starts to emit fluorescence which increases in each cycle proportional to the rate of probe cleavage. Accumulation of PCR products is detected by monitoring the increase in fluorescence of the reporter label (primers are not labeled). Because the cleavage occurs only if the probe hybridizes to the target sequence, the fluorescence detected originates from specific amplification. Assays can be performed using multiple labels with distinct emission wavelengths.

The synthetic specific primers and specific probes of the kit are designed from human genomic HLA-DRB sequences. The term "specific" means that primers and probes comprise a nucleotide sequence totally complementary to an HLA-DRB allelic sequence.

In another particular embodiment, the kit further comprises two primers for glyceraldehydephosphate dehydrogenase (GAPDH) gene with SEQ ID NO: 20 and SEQ ID NO: 21, and one probe for GAPDH with SEQ ID NO: 49, added as a control. This control can be external (in a different tube), but it is preferably internal (in the same tube). The kit can optionally comprise instructions for determining the HLA-DRB genotype of a sample according to kit ingredients.

The genotyping method of the present invention and the kit for performing the method have some clear advantages respect to methods and kits known in the art: post amplification steps are avoided thus saving labor and reducing contamination risk; time required for typing can be shorten to as little as two and a half hours if the initial steps of reagents and sample loading into reaction trays are finally automated; the use of allele-specific probes and the use of more than one probe in a single tube allowed the reduction of the number of PCR tubes, thus further simplifying the PCR typing procedure and resulting in a significant cut in reagents and cost, while typing resolution is increased.

The terms "genotyping" or simply "typing" are used as synonymous in this description and they refer to any testing that reveals the specific alleles inherited by an individual, what is particularly useful for situations in which more than one genotypic combination can produce the same clinical presentation. In the present description, the term "locus" means the position occupied by each HLA gene. "Loci" (plural of locus) for DR antigens include DRA and DRB1-B9. The term "allele" refers to one of two or more alternative forms of a gene, differing in genetic sequence and resulting in observable differences in heritable character (phenotype), and are found at the same place on a chromosome. In the case of HLA DRB1 locus, different alleles of this locus are called DRB1*01, DRB1*02, etc., and these alleles altogether form what is called an "allelic group" in this description.

The genotyping method of the present invention facilitates e.g. the determination of the transplantation compatibility of an individual and his susceptibility for a specific disease. As mentioned above, compared with some HLA typing methods known in the art, the method of the present invention is faster and easier to automate, it gives higher resolution and it reduces sample contamination risks.

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following particular embodiments, drawings and sequence listing are provided by way of illustration and are not intended to be limiting of the present invention.

### BRIEF DESCRIPTION OF THE DRAWINGS

FIG. 1 shows an example of the HLA-DRB typing results of one DNA sample.
FIG. 2 (FIG. 2A-K) shows the fluorescence pattern for interpretation of results.

### DETAILED DESCRIPTION OF PARTICULAR EMBODIMENTS

### Detailed description of figures

In FIG. 1 ΔRn represents the normalized reporter signal minus the baseline signal established in the first cycles of PCR. Rn is the normalized reporter signal, which represents the fluorescence signal of the reporter label divided by the fluorescence signal of the passive reference label (ROX, included in Buffer A). Ct represents the PCR cycle at which an increase in the reporter fluorescence above the baseline signal can be first detected. The Ct for a given amplification curve occurs at the point that the fluorescent signal grows beyond the threshold value of FIG. 1A shows fluorescence amplification plot of FAM probes. ΔRn FAM vs. Ct is plotted. FIG. 1 B shows fluorescence amplification plot of TET probes (GAPDH amplification control). ΔRn TET vs. Ct is represented. FIG. 1C shows fluorescence amplification plot of VIC probes. ΔRn VIC vs. Ct is plotted. Positive probes for this sample were the following: FAM probes of tubes 5, 6, 8, 12, 13 and 15; VIC probes of tubes 3, 4, 7, 12, 13 and 15. The deduced HLA-DRB genotype of this sample was HLA-DRB1*11,15; DRB3; DRB5.

In FIG. 2 (FIG. 2A-K) HLA-DRB specificities detected by each reaction are listed. Horizontally there are the used fluorescent probes, and vertically corresponding specificities. A black filled square indicates a real positive, a cross indicates a non specific positive, "p" indicates a probable positive (not tested), and "pi" indicates a non specific probable positive (not tested).

### Set up of the typing method: construction of a fluorescence pattern

DNA samples: A total of 100 clinical samples from bone marrow or solid organ transplant patients or healthy donors from the inventor's repository of DNA samples were selected to represent common alleles of all serologically defined HLA-DRB specificities in Spanish population. Genomic DNA from these samples was obtained by a standard salting-out method (cf. S.A. Miller et al., "A simple salting out procedure for extracting DNA from human nucleated cells", Nucleic Acids Res. 1988, vol. 16, pp. 1215).

PCR reaction: A set of 16 PCR reaction tubes was used. A total of 19 primers (14 pairs) and 28 fluorogenic probes were used in order to identify all HLA-DRB1, DRB3, DRB4 and DRB5 alleles corresponding to the serological specificities HLA-DR1-16 and DR51-53. Two pairs of primers were used per reaction: one pair for the DRB genes and the other pair for the GAPDH gene to provide the internal positive amplification control. The sequence and localization of the primers are listed in TABLE 1. Three probes with a different fluorescent label, two DRB specific probes labeled in the 5' end with FAM and VIC (except tubes 14 and 16 where only one DRB specific probe was used), and a third GAPDH probe labeled with TET were used for each tube. The sequence, localization, the fluorogenic label and the melting temperatures of the probes are listed in TABLE 2. The design of primers and probes was made from HLA-DRB gene sequences reported in EMBL-EBI Immunogenetics database > IMGT/HLA > Introduction to the IMGT/HLA Sequence Database, cop. 2003. http://www.ebi.ac.uk/imat/hla/intro.html [consulted in 200]. All primers were designed to have a melting temperature at 58-60 °C and all probes at 65-67 °C to ensure that the PCR reactions work under the same conditions. While most probes are attached at the 3' end to the TAMRA quencher label, in some probes a non-fluorescent quencher (MGB probe) was used to be able to use a shorter probe while maintaining the melting temperature. Each PCR reaction was carried out in a final volume of 20 µl containing the following components: Buffer A (TaqMan^{®} 1000 Rxn Gold /Buffer A Pack, Applied Biosystems, Foster City, CA, USA), 0.46 u of AmpliTaq Gold, MgCl₂ [see final concentrations on TABLE 4], 200 µM of each dNTP, primers, a FAM probe, a VIC probe (in TABLE 4, primer mixes and probes used in each tube and their final concentrations are listed), 25 nM of each GAPDH primer, 75 nM of the GAPDH-TET probe and 50-100 ng of template DNA. PCR amplifications were carried out in a 7700 SDS (Applied Biosystems, Foster City, CA, USA) which detected and recorded the fluorescence emission during the amplification process in real-time. After an initial denaturation step at 95 °C for 10 min, the samples were subjected to 40 temperature cycles of a denaturation step at 95 °C for 20 s and an annealing/extension step at 64 °C for 1 min. Taqman probes were from Applied Biosystems, Foster City, CA, USA: FAM (6-carboxy-fluorescein), VIC, TET (6-carboxy-4,7,2',7'-tetrachloro-fluorescein), TAMRA (6-carboxy-N,N,N',N'-tetramethyl-rhodamine) and MGB (DNA minor groove binder).

TABLE 1 shows sequences and position of the 3' end of primers used. Positions of GAPDH primers are referred to the GenBank accession number J04038 sequence. An asterisk indicates that these primers are described in O. Olerup et al., "HLA-DR typing by PCR amplification with sequence specific primers (PCR-SSP) in 2 hours: an alternative to serological DR typing in clinical practice including donor-recipient matching in cadaveric transplantation", Tissue Antigens, 1992, vol. 39, pp. 225-35. The symbol # indicates that the primer is described in K. Kotsch et al., "Sequencing of HLA class II genes based on the conserved diversity of the non coding regions: sequencing based typing of HLA-DRB genes", Tissue Antigens 1999, vol. 53, pp. 486-97. RN indicates CAS Registry Number. In degenerated primer sequences, symbol Y indicates C or T, S indicates G or C, and K indicates G or T.

Construction of a fluorescence pattern: The pattern for the interpretation of the positive signals was deduced comparing the sequences of primers and probes used in each reaction and the sequences of all published alleles. In some cases the predicted results were modified by the observed results obtained with the samples used in the set up of the method. FIG. 2 shows the resulting fluorescence pattern.

### Validation of the genotyping method

DNA samples and PCR reaction: Two hundred clinical samples from bone marrow or solid organ transplant patients or healthy donors were selected at random to validate the method. Genomic DNA from these samples was obtained by a standard salting-out method. PCR reaction for these samples was performed as in the set up of typing method described above. An example for the HLA-DRB typing of one DNA sample by the new method is given in FIG. 1.

### Data analysis and interpretation

For HLA-DRB fluorogenic typing, PCR amplification of one sample was performed with all 16 primers and probes mixes in parallel. The presence of a PCR product was associated with a significant increase in the fluorescence emission of the corresponding reporter label. In TABLE 5, HLA-DRB specificities detected by each reaction are listed.

### Comparison with other genotyping methods

Independently, all samples were typed for HLA-DRB by a PCR-based method, such as PCR-SSO, PCR-SSP or sequence-based typing (SBT). The two hundred samples analyzed to validate the technique gave results that were concordant in all cases with HLA types previously obtained by conventional DNA typing methods, as TABLE 6 shows. An important reduction in the proportion of ambiguous results was observed by the new method as compared to standard low resolution PCR-SSP or PCR-SSO. With the new method, intermediate-high resolution typing was achieved in some cases.

**TABLE 1. Sequences and position of the 3' end of primers used.**

| Primer | Sequence | Position | Reference |
|---|---|---|---|
| E02* | TCCTGTGGCAGCCTAAGAGG | exon 2-126 | RN 213397-50-3 SEQ ID NO: 1 |
| E04* | ACGTTTCTTGGAGCAGGTTAAAC | exon 2-124 | RN 590447-87-3 SEQ ID NO: 2 |
| E2-100 FW | GCACGTTTCTTGGAGTACTCTACG | exon 2-123 | RN 149782-49-0 SEQ ID NO: 3 |
| E2-144C RV | TCCGTCCCGTTGAAGAAAT | exon 2-134 | SEQ ID NO: 4 |
| E2-201 FW | CGCTTCGACAGCGACGT | exon 2-219 | RN 134193-51-4 Acc. No. BD104297 SEQ ID NO: 5 |
| E2-201 RV | CACGTCGCTGTCGAAGCG | exon 2-201 | SEQ ID NO: 6 |
| E2-210 RV | TACTCCCCCACGTCGCTG | exon 2-210 | SEQ ID NO: 7 |
| E2-322 RV | CYCCGTAGTTGTGTCTGCA | exon 2-322 | SEQ ID NO: 8 |
| 11-04 FW | CTTCTGTAACCGGATCGTTCTT | intron 1-475 | SEQ ID NO: 9 |
| 11-07 FW | GTCAGTGTTTTTCCCGGAGA | intron 1-450 | SEQ ID NO: 10 |
| I1-Com FW | TTCGTGTCCCCACAGCAC | exon 2-103 | RN 451718-32-4 SEQ ID NO: 11 |
| I2-15 RV | CCGCGGCCATGCTCAC | intron 2-1 | SEQ ID NO: 12 |
| I2-1Com RV | CGCGCCGCGCTCAC | intron 2-1 | SEQ ID NO: 13 |
| 12-45 RV | CACACACACACACACACTCAGATTC | intron 2-43 | SEQ ID NO: 14 |
| I2-RB7# | ACACACACACTCAGATTCTCC | intron 2-40 | RN 259466-27-8 Acc. No. AX001239 SEQ ID NO: 15 |
| DRB3 102-FW | ACGTTTCTYGSAGCTGYKTAA | exon 2-102 | SEQ ID NO: 16 |
| DRB4 115-FW | CAGGCTAAGTGTGAGTGTCATTTCC | exon 2-115 | SEQ ID NO: 17 |
| DRB4 328-RV | CTCTCCACAACCCCGTAGTTGTA | exon 2-328 | RN 515893-57-9 SEQ ID NO: 18 |
| DRB5 102-FW | A(C/T)GTTTCTTGCAGCAGGATAAGTA | exon 2-102 | SEQ ID NO: 19 |
| GAPDH FW | AAGGTGGTGAAGCAGGCGT | exon 8-4476 | SEQ ID NO: 20 |
| GAPDH Intron H | TCTGAGCCAGCCACCAGAG | intron H-4671 | SEQ ID NO: 21 |

**TABLE 2. Sequences, fluorogenic labels, melting temperatures and position of the fluorogenic probes used. All HLA-DRB probes are placed in exon 2. Position of GAPDH probe is referred to the GenBank accession number J04038 sequence. An asterisk means MGB probe.**

| N° | Probe | Sequence | Label | Tm (°C) | Position | SEQ ID NO: |
|---|---|---|---|---|---|---|
| 1F | EVH | CACTCATGTTTAACCTGCTCCAAGAAACG | FAM | 66.3 | 130-103 | 22 |
| 1V | WQG | TTACCCTGCCACAGGAAACGTGC | VIC | 66 | 122-100 | 23 |
| 2F | K-D | AGAAATGACACTCAAACTTATCCTGCTTCAAGA | FAM | 65.8 | 139-107 | 24 |
| 2V | EEV | CTCAAACTTAACCTCC | VIC* | 66.2 | 129-114 | 25 |
| 3F | WQLF | ATGACATTCAAACTTAAGCTGCCACAAGAAA | FAM | 66.2 | 135-105 | 26 |
| 3V | EYSTS | TCAGACGTAGAGTACTC | VIC* | 66.5 | 128-113 | 27 |
| 4F | EYSTG | AGAAATAACACTCACCCGTAGAGTACTCCAAGAA | FAM | 65.6 | 138-106 | 28 |
| 4V | P-R | ACTCCCTCTTAGGCTGCCACAGGA | VIC | 65.2 | 129-107 | 29 |
| 5F | EQA | CGGCCCGCGCCTGCT | FAM | 66.6 | 307-293 | 30 |
| 5V, 13V | KDFL | TGTCTTCCAGGAAGTCCTTCTGGCTG | VIC | 66.6 | 298-273 | 31 |
| 6F | FDYF | TACTTCCATAACCAAGAGGAG | FAM* | 66.6 | 175-195 | 32 |
| 6V, 15V | YFHN | TACTTCCATAACCAGGAGG | VIC* | 66.4 | 175-193 | 33 |
| 7F | AEH | TGTTCCAGTGCTCCGCAGCAG | FAM | 65.6 | 271-251 | 34 |
| 7V | RPDE | TCCAGTACTCCTCATCAGGCCGC | VIC | 65.5 | 271-249 | 35 |
| 8F | GEFR | CGCCCGGAACTCCCCCA | FAM | 66.1 | 235-218 | 36 |
| 8V | EQR | CGGCCCGCCTCTGCTCC | VIC | 66.2 | 308-291 | 37 |
| 9F | EDER | CGGCCCGCTCGTCTTCCA | FAM | 66.5 | 307-290 | 38 |
| 9V | EDKR | CGCGGCCCGCTTGTCTTC | VIC | 65.9 | 308-292 | 39 |
| 10F | VAES | TCCAGGACTCGGCGACAGGC | FAM | 66.7 | 271-251 | 40 |
| 10V | PSAE | TACTCGGCGCTAGGCCGCC | VIC | 66.4 | 266-248 | 41 |
| 11F | RALV | TAGGTGTCCACCAGGGCCCG | FAM | 66 | 320-301 | 42 |
| 11V | EQK-Q | CCGGCCCCGCTTCTGCT | VIC | 65.5 | 309-293 | 43 |
| 12F | VGE | TGAAGCTCTCACCAACCCCGTAGTTG | FAM | 66 | 355-330 | 44 |
| 12V | VVE | TGAAGCTCTCCACAACCCCGTAGTTG | VIC | 66 | 355-330 | 45 |
| 13F | KDIL | TCCAGGATGTCCTTC | FAM* | 66.9 | 294-279 | 46 |
| 14V | RAAV | AGGTGTCCACCTCGGCCCG | VIC | 66.4 | 319-301 | 47 |
| 16F | PDAE | TTCCAGTACTCAGCGTCAGGCCG | FAM | 66.4 | 272-250 | 48 |
| | GAPDHex8 | CAAGGGCATCCTGGGCTACACTGA | TET | 67.1 | 4490-4513 | 49 |

**TABLE 3. Preparation of primers mixtures. The final concentration of mixtures was 12.5 µM except mix 10 at 50 µM. Symbol indicates a concentration for that primer of 1.56 µM; symbol # indicates 5 µM, and symbol ∞ indicates 25 nM.**

| Mix | Sense Primers | Antisense Primers |
|---|---|---|
| 1 | I1-04 FW | E2-144C RV |
| | I1-07 FW | |
| 2 | I1-Com FW | E2-210 RV |
| 3 | I1-Com FW | E2-201 RV |
| 4 | E02 | E2-322 RV |
| 7 | E2-100 FW | E2-201 RV |
| 8 | E2-100 FW | E2-322 RV |
| 5 | E2-201 FW | I2-45 RV |
| 12 | E2-100 FW* | I2-1Com RV* |
| | E02* | I2-15 RV* |
| 9 | E04 | 12RB7 |
| 10 | DRB5 102-FW^{#} | E2-322 RV^{#} |
| | DRB3 102-FW^{#} | |
| 11 | DRB4 115-FW | DRB4 327-RV |
| GAPDH | GAPDH FW^{∞} | GAPDH Intron H^{∞} |

**TABLE 4. Reagents amounts for the PCR (in µl)**

| Tubes | 1 | 2 | 3 | 4 | 5 | 6 | 7 | 8 | 9 | 10 | 11 | 12 | 13 | 14 | 15 | 16 |
|---|---|---|---|---|---|---|---|---|---|---|---|---|---|---|---|---|
| primer mix | 1 | 2 | 3 | 3 | 4 | 7 | 8 | 5 | 5 | 5 | 5 | 5 | 12 | 9 | (50 µM) | 11 |
| probe FAM | 1F | 2F | 3F | 4F | 5F | 6F | 7F | 8F | 9F | 10F | 11F | 12F | 13F | | 15F | 16F |
| probe VIC | 1V | 2V | 3V | 4V | 5V | 6V | 7V | 8V | 9V | 10V | 11V | 12V | 13V | 14V | 15V | |
| buffer A 10x | 2 | 2 | 2 | 2 | 2 | 2 | 2 | 2 | 2 | 2 | 2 | 2 | 2 | 2 | 2 | 2 |
| MgCl₂ 25 mM | 4 | 2,4 | 4 | 3,2 | 2,4 | 2,4 | 3,6 | 2,4 | 2,4 | 3,2 | 1,6 | 3,2 | 1,6 | 1,6 | 2,4 | 2,4 |
| dNTPs | 2 | 2 | 2 | 2 | 2 | 2 | 2 | 2 | 2 | 2 | 2 | 2 | 2 | 2 | 2 | 2 |
| primer mix 12,5 µM | 1,25 | 1,25 | 1,25 | 1,25 | 1,25 | 1,25 | 1,25 | 1,25 | 1,25 | 1,25 | 1,25 | 1,25 | 2,5 | 1,25 | 2 | 1,25 |
| FAM 5 µM | 0,6 | 1 | 1 | 1 | 0,6 | 1 | 0,6 | 0,6 | 1 | 0,4 | 0,4 | 0,7 | 0,5 | | 0,8 | 0,6 |
| VIC 5 µM | 0,6 | 1 | 1 | 0,8 | 0,6 | 1 | 0,6 | 0,8 | 1 | 1 | 0,4 | 0,7 | 1 | 0,6 | 1 | |
| primers GAPDH 1 µM | 0,5 | 0,5 | 0,5 | 0,5 | 0,5 | 0,5 | 0,5 | 0,5 | 0,5 | 0,5 | 0,5 | 0,5 | 0,5 | 0,5 | 0,5 | 0,5 |
| GAPDH-TET 15 µM | 0,2 | 0,2 | 0,2 | 0,2 | 0,2 | 0,2 | 0,2 | 0,2 | 0,2 | 0,2 | 0,2 | 0,2 | 0,2 | 0,2 | 0,2 | 0,2 |
| H₂O | 3,85 | 4,65 | 3,05 | 4,05 | 5,45 | 4,65 | 4,25 | 5,25 | 4,65 | 4,45 | 6,65 | 4,45 | 4,7 | 6,85 | 4,1 | 6,05 |
| total volume of reagents | 15 | 15 | 15 | 15 | 15 | 15 | 15 | 15 | 15 | 15 | 15 | 15 | 15 | 15 | 15 | 15 |
| Master Mix volume of reagents for PCR | 5 | 5 | 5 | 5 | 5 | 5 | 5 | 5 | 5 | 5 | 5 | 5 | 5 | 5 | 5 | 5 |
| final volume per tube | 20 | 20 | 20 | 20 | 20 | 20 | 20 | 20 | 20 | 20 | 20 | 20 | 20 | 20 | 20 | 20 |

**TABLE 5. Primers and probes used in each tube and the specificities detected by each reaction.**

| Tube | Primer Mix | Probes | HLA-DRB specificity |
|---|---|---|---|
| 1 | 1 | 1F | 04 |
| | | 1V | 07 |
| 2 | 2 | 2F | 09 |
| | | 2V | 10 |
| 3 | 3 | 3F | 01 |
| | | 3V | 03, 0820, 1101-04/..., 1301-16/..., 1401-03/... |
| 4 | 3 | 4F | 0801-19/..., 1105, 12, 1317, 1404/... |
| | | 4V | 15, 16 |
| 5 | 4 | 5F | 15 |
| | | 5V | 1504, 1601 /02/... |
| 6 | 7 | 6F | 0317, 0801-08/..., 1101-08/..., 1303/04/..., 1425/... |
| | | 6V | 0301-16/..., 0809/21, 1109/..., 12, 1301-02/05-06/..., 1401-24/... |
| 7 | 8 | 7F | 0310, 0808, 1343/..., 1401/04/... |
| | | 7V | 0308, 11, 1204, 1411 |
| 8 | 5 | 8F | 0301/04/..., 0817, 1101-16/..., 12, 1301-02/04-06/..., 1417/..., 1501-06/... |
| | | 8V | 0101-02/04-05/..., 0403-05/06-0701/08/..., 09, 1126, 1344, 1401/02/04-11/... |
| 9 | 5 | 9F | 0103, 0402, 1102-03/..., 1301-02/04/..., 1416, 1510 |
| | | 9V | 1303/10/... |
| 10 | 5 | 10F | 0701-05/07, 090102, 1201-03/... |
| | | 10V | 0312, 0405/09-12/..., 0801/03/05-06/..., 1303-04/..., 1413, 1512 |
| 11 | 5 | 11F | 0412/..., 0801-04/06-17/..., 1123/25, 1313/..., 1403/..., 1604 |
| | | 11V | 0301-10/..., 0422, 1107 |
| 12 | 5 | 12F | 0101/03/..., 0302/..., 0401/05/07-09/..., 07, 0801-03/05/07-09/..., 09, 10, |
| | | 12V | 0102/..., 0301/03-04/..., 0402-04/06/10-13/..., 0804/06/..., 1102-04/..., |
| 13 | 12 | 13F | 0803/10/..., 1102/..., 1203-06/08, 1301-04/06/..., 1416/..., 1501-03/... |
| | | 13V | 0801-02/04-09/..., 1101/03-06/..., 1202, 1305/07/..., 1415/..., 1504 |
| 14 | 9 | 14V | 0403/06-07/11/... |
| 15 | 10 | 15F | DRBS*0101-09, 02 |
| | | 15V | DRB3 |
| 16 | 11 | 16F | DRB4*01, 02 |

**TABLE 6. Concordance of results from the new genotyping method and other typing methods used. Number of samples for each type of analysis is indicated.**

| allelic group | PCR-SSO and PCR-SBT typing | new genotyping method | Concordance |
|---|---|---|---|
| DRB1*01 | 38 | 38 | 100% |
| DRB1*03 | 55 | 55 | 100% |
| DRB1*04 | 44 | 44 | 100% |
| DRB1*07 | 69 | 69 | 100% |
| DRB1*08 | 10 | 10 | 100% |
| DRB1*09 | 8 | 8 | 100% |
| DRB1*10 | 3 | 3 | 100% |
| DRB1*11 | 62 | 62 | 100% |
| DRB1*12 | 11 | 11 | 100% |
| DRB1*13 | 47 | 47 | 100% |
| DRB1*14 | 14 | 14 | 100% |
| DRB1*15 | 31 | 31 | 100% |
| DRB1*16 | 8 | 8 | 100% |
| DRB3 | 165 | 165 | 100% |
| DRB4 | 109 | 109 | 100% |
| DRB5 | 37 | 37 | 100% |

## Claims

1. A method for determining the genotype of the loci DRB of human leukocyte antigen (HLA-DRB) from a nucleic acid sample, comprising the steps of:
(i) amplifying any nucleic acid that comprises exon 2 of at least one of the genes of the DRB loci by real-time polymerase chain reaction (PCR);
(ii) detecting fluorescence signals by fluorescently labelled nucleotide probes during the amplification carried out in step (i), under conditions where said probes specifically bind to the amplified nucleic acid sequences; and
(iii) comparing more than one signal detected in step (ii) with an experimentally defined fluorescence pattern; said pattern having been established by an initial definition based on theoretical comparison of the sequences of nucleotide probes of step (ii) with the sequences of the different alleles of the selected genes of step (i), followed by a definitive definition based on an experimental assessment of which of the theoretical signals are actually positive (black squares in FIG. 2) and which are actually negative (white squares in FIG. 2);
wherein steps (i) and (ii) are performed in the same tube and without any further manipulation of the sample.

2. The method according to claim 1, wherein the genes of the DRB loci in step (ii) are selected from the group consisting of DRB1, DRB3, DRB4 and DRB5.

3. The method according to claim 2, wherein the amplifying step (i) is carried out with two or more primers which are single strand nucleic acids having a length from 10 to 30 nucleotides and comprising sequences complementary to sequences of a region located along intron 1-exon 2-intron 2 of at least one of the genes selected from the group consisting of DRB1, DRB3, DRB4 and DRB5.

4. The method according to claim 3, wherein length of primers is from 14 to 24 nucleotides.

5. The method according to claim 4, wherein primer sequences are selected from the group consisting of SEQ ID NO: 1-19.

6. The method according to any of the claims 2-5 wherein the detecting step (ii) is carried out with one or more probes which are single strand nucleic acids having a length from 10 to 45 nucleotides and which comprise sequences complementary to sequences of a region located along exon 2 of at least one of the genes selected from the group consisting of DRB1, DRB3, DRB4 and DRB5.

7. The method according to claim 6, wherein length of probes is from 13 to 34 nucleotides.

8. The method according to claim 7, wherein probe sequences are selected from the group consisting of SEQ ID NO: 22-48.

9. The method according to claim 7, wherein probe sequences are selected from the complementary sequences of the group consisting of SEQ ID NO: 22-48.

10. The method according to any of the claims 6-9, wherein probes are labelled with fluorochromes of type reporter label-quencher label, and probes are degraded due to the 5'->3' exonuclease activity of DNA polymerase.

11. The method according to any of the previous claims, wherein two primers for glyceraldehydephosphate dehydrogenase (GAPDH) gene with SEQ ID NO: 20 and SEQ ID NO: 21, and one probe for GAPDH with SEQ ID NO: 49 are added as a control.

12. A kit for determining the HLA-DRB (loci DRB of human leukocyte antigen) genotype from a nucleic acid sample by performing the method of claim 1, comprising:
(i) primers for amplifying nucleic acids of at least one of the genes of the DRB loci by real-time polymerase chain reaction (PCR);
(ii) fluorescently labelled nucleotide probes for detecting fluorescence signals during the amplification; and
(iii) experimentally defined fluorescence patterns for comparing the detected signals, said patterns having been established by an initial definition based on theoretical comparison of the sequences of nucleotide probes with the sequences of the different alleles of HLA-DRB loci, followed by a definitive definition based on an experimental assessment of which of the theoretical signals are actually positive (black squares in FIG. 2) and which are actually negative (white squares in FIG. 2).

13. The kit according to claim 12, wherein the genes of the DRB loci are selected from the group consisting of DRB1, DRB3, DRB4 and DRB5.

14. The kit according to claim 13, wherein primers are single strand nucleic acids which have a length from 10 to 30 nucleotides and which comprise sequences complementary to sequences of a region located along intron 1-exon 2-intron 3 of at least one of the genes selected from the group consisting of DRB1, DRB3, DRB4 and DRB5.

15. The kit according to claim 14, wherein length of primers is from 14 to 24 nucleotides.

16. The kit according to claim 15, wherein primer sequences are selected from the group consisting of SEQ ID NO: 1-19.

17. The kit according to any of the claims 13-16, wherein probes are single strand nucleic acids which have a length from 10 to 45 nucleotides and which comprise sequences complementary to sequences of a region located along exon 2 of at least one of the genes selected from the group consisting of DRB1, DRB3, DRB4 and DRB5.

18. The kit according to claim 17, wherein length of probes is from 13 to 34 nucleotides.

19. The kit according to claim 18, wherein probe sequences are selected from the group consisting of SEQ ID NO: 22-48.

20. The kit according to claim 18, wherein probe sequences are selected from the complementary sequences of the group consisting of SEQ ID NO: 22-48.

21. The kit according to any of the claims 17-20, wherein probes are labelled with fluorochromes of type reporter label-quencher label, and probes are degraded due to the 5'->3' exonuclease activity of DNA polymerase.

22. The kit according to any of the claims 12-21, wherein two primers for glyceraldehydephosphate dehydrogenase (GAPDH) gene with SEQ ID NO: 20 and SEQ ID NO: 21, and one probe for GAPDH with SEQ ID NO: 49 are added as a control.

23. A kit for determining the HLA-DRB (loci DRB of human leukocyte antigen) genotype from a nucleic acid sample by performing the method of claim 1, comprising:
(i) primers for amplifying nucleic acids of at least one of the genes of the DRB loci by real-time polymerase chain reaction (PCR), these primers with sequences selected from the group consisting of SEQ ID NO: 1-19;
(ii) fluorescently labelled nucleotide probes for detecting fluorescence signals during the amplification, these probes with sequences selected from the group consisting of SEQ ID NO: 22-48;
(iii) two primers for glyceraldehydephosphate dehydrogenase (GAPDH) gene with SEQ ID NO: 20 and SEQ ID NO: 21, and one probe for GAPDH with SEQ ID NO: 49 added as a control; and
(iv) experimentally defined fluorescence patterns for comparing the detected signals, these patterns having been established by an initial definition based on theoretical comparison of the sequences of nucleotide probes with the sequences of the different alleles of HLA-DRB loci, followed by a definitive definition based on an experimental assessment of which of the theoretical signals are actually positive (black squares in FIG. 2) and which are actually negative (white squares in FIG. 2).


SEQUENCE LISTING

<110>  Universitat Autonoma de Barcelona

<120>  Method and kit for HLA-DRB genotyping based on real-time PCR

<130>  P559EP00

<150>  P200400010
<151>  2003-12-17

<150>  PCT/ES2004/070104
<151>  2004-12-03

<160>  49

<170>  PatentIn version 3.3

<210>  1
<211>  20
<212>  DNA
<213>  Homo sapiens

<400>  1
tcctgtggca gcctaagagg                                                    20


<210>  2
<211>  23
<212>  DNA
<213>  Homo sapiens

<400>  2
acgtttcttg gagcaggtta aac                                                23


<210>  3
<211>  24
<212>  DNA
<213>  Homo sapiens

<400>  3
gcacgtttct tggagtactc tacg                                               24


<210>  4
<211>  19
<212>  DNA
<213>  Homo sapiens

<400>  4
tccgtcccgt tgaagaaat                                                     19


<210>  5
<211>  17
<212>  DNA
<213>  Homo sapiens

<400>  5

cgcttcgaca gcgacgt                                                    17

<210>  6
<211>  18
<212>  DNA
<213>  Homo sapiens

<400>  6
cacgtcgctg tcgaagcg                                                   18

<210>  7
<211>  18
<212>  DNA
<213>  Homo sapiens

<400>  7
tactccccca cgtcgctg                                                   18

<210>  8
<211>  19
<212>  DNA
<213>  Homo sapiens

<400>  8
cyccgtagtt gtgtctgca                                                  19

<210>  9
<211>  22
<212>  DNA
<213>  Homo sapiens

<400>  9
cttctgtaac cggatcgttc tt                                              22

<210>  10
<211>  20
<212>  DNA
<213>  Homo sapiens

<400>  10
gtcagtgttt ttcccggaga                                                 20

<210>  11
<211>  18
<212>  DNA
<213>  Homo sapiens

<400>  11
ttcgtgtccc cacagcac                                                   18

<210>  12
<211>  16
<212>  DNA

<213>  Homo sapiens

<400>  12
ccgcggccat gctcac                                                        16


<210>  13
<211>  14
<212>  DNA
<213>  Homo sapiens

<400>  13
cgcgccgcgc tcac                                                          14


<210>  14
<211>  25
<212>  DNA
<213>  Homo sapiens

<400>  14
cacacacaca cacacactca gattc                                              25


<210>  15
<211>  21
<212>  DNA
<213>  Homo sapiens

<400>  15
acacacacac tcagattctc c                                                  21


<210>  16
<211>  21
<212>  DNA
<213>  Homo sapiens

<400>  16
acgtttctyg sagctgykta a                                                  21


<210>  17
<211>  25
<212>  DNA
<213>  Homo sapiens

<400>  17
caggctaagt gtgagtgtca tttcc                                              25


<210>  18
<211>  23
<212>  DNA
<213>  Homo sapiens

<400>  18
ctctccacaa ccccgtagtt gta                                                23

<210>  19
<211>  24
<212>  DNA
<213>  Homo sapiens

<400>  19
aygtttcttg cagcaggata agta                                              24


<210>  20
<211>  19
<212>  DNA
<213>  Homo sapiens

<400>  20
aaggtggtga agcaggcgt                                                    19


<210>  21
<211>  19
<212>  DNA
<213>  Homo sapiens

<400>  21
tctgagccag ccaccagag                                                    19


<210>  22
<211>  29
<212>  DNA
<213>  Homo sapiens

<400>  22
cactcatgtt taacctgctc caagaaacg                                         29


<210>  23
<211>  23
<212>  DNA
<213>  Homo sapiens

<400>  23
ttaccctgcc acaggaaacg tgc                                               23


<210>  24
<211>  33
<212>  DNA
<213>  Homo sapiens

<400>  24
agaaatgaca ctcaaactta tcctgcttca aga                                    33


<210>  25
<211>  16
<212>  DNA
<213>  Homo sapiens

<400>  25

ctcaaactta acctcc                                                    16


<210>  26
<211>  31
<212>  DNA
<213>  Homo sapiens

<400>  26
atgacattca aacttaagct gccacaagaa a                                   31


<210>  27
<211>  17
<212>  DNA
<213>  Homo sapiens

<400>  27
tcagacgtag agtactc                                                   17


<210>  28
<211>  34
<212>  DNA
<213>  Homo sapiens

<400>  28
agaaataaca ctcacccgta gagtactcca agaa                                34


<210>  29
<211>  24
<212>  DNA
<213>  Homo sapiens

<400>  29
actccctctt aggctgccac agga                                           24


<210>  30
<211>  15
<212>  DNA
<213>  Homo sapiens

<400>  30
cggcccgcgc ctgct                                                     15


<210>  31
<211>  26
<212>  DNA
<213>  Homo sapiens

<400>  31
tgtcttccag gaagtccttc tggctg                                         26


<210>  32
<211>  21
<212>  DNA

<213>  Homo sapiens

<400>  32
tacttccata accaagagga g                                                21


<210>  33
<211>  19
<212>  DNA
<213>  Homo sapiens

<400>  33
tacttccata accaggagg                                                   19


<210>  34
<211>  21
<212>  DNA
<213>  Homo sapiens

<400>  34
tgttccagtg ctccgcagca g                                                21


<210>  35
<211>  23
<212>  DNA
<213>  Homo sapiens

<400>  35
tccagtactc ctcatcaggc cgc                                              23


<210>  36
<211>  17
<212>  DNA
<213>  Homo sapiens

<400>  36
cgcccggaac tccccca                                                     17


<210>  37
<211>  17
<212>  DNA
<213>  Homo sapiens

<400>  37
cggcccgcct ctgctcc                                                     17


<210>  38
<211>  18
<212>  DNA
<213>  Homo sapiens

<400>  38
cggcccgctc gtcttcca                                                    18

<210>  39
<211>  18
<212>  DNA
<213>  Homo sapiens

<400>  39
cgcggcccgc ttgtcttc                                                    18


<210>  40
<211>  20
<212>  DNA
<213>  Homo sapiens

<400>  40
tccaggactc ggcgacaggc                                                  20


<210>  41
<211>  19
<212>  DNA
<213>  Homo sapiens

<400>  41
tactcggcgc taggccgcc                                                   19


<210>  42
<211>  20
<212>  DNA
<213>  Homo sapiens

<400>  42
taggtgtcca ccagggcccg                                                  20


<210>  43
<211>  17
<212>  DNA
<213>  Homo sapiens

<400>  43
ccggccccgc ttctgct                                                     17


<210>  44
<211>  26
<212>  DNA
<213>  Homo sapiens

<400>  44
tgaagctctc accaaccccg tagttg                                           26


<210>  45
<211>  26
<212>  DNA
<213>  Homo sapiens

<400>  45

tgaagctctc cacaaccccg tagttg                                          26

<210>  46
<211>  15
<212>  DNA
<213>  Homo sapiens

<400>  46
tccaggatgt ccttc                                                     15

<210>  47
<211>  19
<212>  DNA
<213>  Homo sapiens

<400>  47
aggtgtccac ctcggcccg                                                 19

<210>  48
<211>  23
<212>  DNA
<213>  Homo sapiens

<400>  48
ttccagtact cagcgtcagg ccg                                            23

<210>  49
<211>  24
<212>  DNA
<213>  Homo sapiens

<400>  49
caagggcatc ctgggctaca ctga                                           24